# FASCICULE DE BREVET EUROPEEN

(11) **EP 1 165 565 B1**
(45) Date de publication et mention de la délivrance du brevet: **04.06.2003**
(21) Numéro de dépôt: 00907714.0
(22) Date de dépôt: 24.02.2000
(51) Int. Cl.: C07D 491/22, A61K 31/47

(54) **ANALOGUES OPTIQUEMENT PURS DE LA CAMPTOTHECINE**
OPTISCH REINE ANALOGE DER CAMPTOTHECINE
OPTICALLY PURE CAMPTOTHECIN ANALOGUES

(30) Priorité: 26.02.1999 FR 9902398
(43) Date de publication de la demande: 02.01.2002
(60) Demande divisionnaire: 03075924.5
(73) Titulaire: SOCIETE DE CONSEILS DE RECHERCHES ET D'APPLICATIONS SCIENTIFIQUES (S.C.R.A.S.), 75016 Paris (FR)
(72) Inventeur: LAVERGNE, Olivier, F-91120 Palaiseau (FR); BIGG, Dennis, F-91190 Gif-sur-Yvette (FR); LANCO, Christophe, F-91410 Dourdan (FR); ROLLAND, Alain, F-91120 Palaiseau (FR)
(74) Mandataire: Bourgouin, André
(86) Numéro de dépôt international: FR0000461
(87) Numéro de publication internationale: WO00050427

(56) Documents cités:
- WO-A-97/00876
- WO-A-98/28304
- WO-A-98/28305
- WO-A-98/35940
- WO-A-99/11646
- FR-A- 2 757 514
- FR-A- 2 757 515
- O. LAVERGNE ET AL.: BIOORG. MED. CHEM. LETTERS, vol. 7, no. 17, 1997, pages 2235-2238, XP004136419
- EJIMA A ET AL: "ANTITUMOR AGENTS. V. SYNTHESIS AND ANTILEUKEMIC ACTIVITY OF E-RING -MODIFIED (RS)-CAMPTOTHECIN ANALOGUES" CHEMICAL AND PHARMACEUTICAL BULLETIN, vol. 40, no. 3, 1 mars 1992 (1992-03-01), pages 683-688, XP000653713 ISSN: 0009-2363

## Description

La camptothécine est un composé naturel qui a été isolé pour la première fois des feuilles et de l'écorce de la plante chinoise appelée *camptotheca acuminata* (voir Wall et ses collaborateurs, J. Amer. Chem. Soc. 88:3888 (1966)). La camptothécine est un composé pentacydique constitué d'un fragment indolizino[1,2-b]quinoléine fusionné avec une α-hydroxylactone à six chaînons. Le carbone en position 20, qui porte le groupe α-hydroxy, est asymétrique et confère à la molécule un pouvoir rotatoire. La forme naturelle de la camptothécine possède la configuration absolue "S" au carbone 20 et répond à la formule suivante :

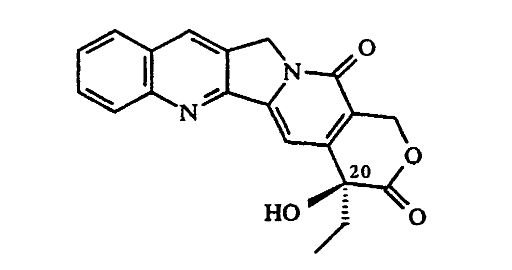

La camptothécine présente une activité anti-proliférative dans plusieurs lignées cellulaires cancéreuses, comprenant les lignées cellulaires de tumeurs humaines du colon, du poumon et du sein (Suffness, M. et collaborateurs : The Alkaloids Chemistry and Pharmacology, Bross, A., ed., Vol. 25, p. 73 (Academic Press, 1985)). On suggère que l'activité anti-proliférative de la camptothécine est en relation avec son activité inhibitrice de la topoisomérase I de l'ADN.

Il avait été indiqué que l'α-hydroxylactone était une exigence absolue à la fois pour l'activité *in vivo* et *in vitro* de la camptothécine (Camptothecins: New Anticancer Agents, Putmesil, M., et ses collaborateurs, ed., p. 27 (CRC Press, 1995) ; Wall, M. et ses collaborateurs, Cancer Res. 55:753 (1995) ; Hertzberg et ses collaborateurs, J. Med. Chem. 32:715 (1982) et Crow et ses collaborateurs, J. Med. Chem. 35:4160 (1992)). La demanderesse a par la suite mis au point une nouvelle classe d'analogues de la camptothécine, dans lesquels une β-hydroxylactone remplace l'α-hydroxylactone naturelle de la camptothécine (cf. les demandes de brevet WO 97/00876, WO 98/28304 et WO 98/28305).

La présente demande concerne de nouveaux analogues β-hydroxylactoniques de la camptothécine dont l'activité biologique, exprimée par exemple en termes de concentrations inhibitrices de la prolifération de colonies cellulaires tumorales, est, de façon inattendue, supérieure à l'activité des composés déjà connus. L'invention a également pour objet, à titre de médicaments, les composés précédemment cités ainsi que les compositions pharmaceutiques les contenant. L'invention concerne également un procédé de préparation de composés selon l'invention.

L'invention a donc pour objet les composés de formule générale I_{A} 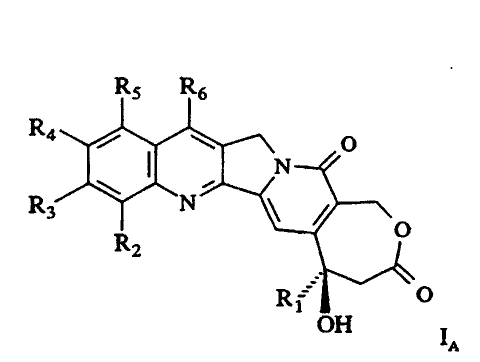 dans laquelle
- R₁: représente un radical alkyle inférieur ;
- R₂, R₃, R₄ et R₅: représentent, indépendamment, un atome d'hydrogène, un radical halo ou -OSO₂R₁₀ ;
- R₆: représente un radical -(CH₂)ₘSiR₇R₈R₉;
- R₇, R₈ et R₉: représentent, indépendamment, un radical alkyle inférieur ;
- R₁₀: représente un radical alkyle inférieur éventuellement substitué par un ou plusieurs radicaux halo identiques ou différents, ou aryle éventuellement susbtitué par un ou plusieurs radicaux alkyle inférieur identiques ou différents ;
- m: est un nombre entier compris entre 0 et 6 ;
ou les sels d'addition de ces derniers.

Dans les définitions indiquées ci-dessus, l'expression halo représente le radical fluoro, chloro, bromo ou iodo, et de préférence chloro, fluoro ou bromo. Par radical alkyle inférieur, on entend dans la présente demande un radical alkyle linéaire ou ramifié comptant de 1 à 6 atomes de carbone tels que les radicaux méthyle, éthyle, propyle, isopropyle, butyle, isobutyle, sec-butyle et tert-butyle, pentyle, isopentyle, neopentyle, hexyle ou isohexyle. Le terme aryle désigne un composé hydrocarboné mono-, di ou tricyclique avec au moins un cycle aromatique, chaque cycle contenant au maximum 7 chaînons, comme par exemple phényle, naphtyle, anthracyle, biphényle ou indényle.

Des composés particulièrement préférés selon l'invention sont ceux pour lesquels R₁ représente un radical éthyle ainsi que ceux pour lesquels R₃ représente un radical halo et en particulier fluoro.

De préférence, les composés de formule I_{A} répondent à l'une des formules suivantes :
(5R)-5-éthyl-9,10-difluoro-5-hydroxy-12-(2-triméthylsilyléthyl)-4,5,13,15-tétrahydro-1*H*,3*H*-oxépino [3',4':6,7]indolizino[1,2-*b*]quinoléine-3,15-dione ;
(5*R*)-5-éthyl-5-hydroxy-12-(2-triméthylsilyléthyl)-4,5,13,15-tétrahydro-1*H*,3*H*-oxépino [3',4':6,7] indolizino[1,2-*b*]quinoléine-3,15-dione.

Les composés de formule générale (I_{A}) peuvent être préparés de la façon suivante :
- on couple un produit de formule générale (M) 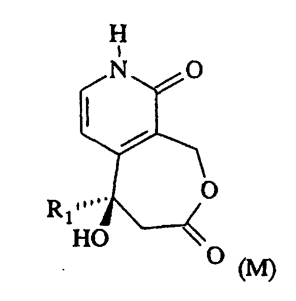 dans laquelle R₁ a la signification indiquée ci-dessus, avec un composé de formule (N) 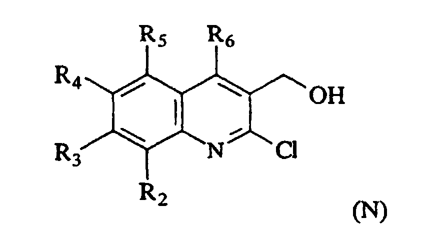 dans laquelle R₂, R₃, R₄, R₅ et R₆ ont la signification indiquée ci-dessus, pour donner le composé de formule (O) 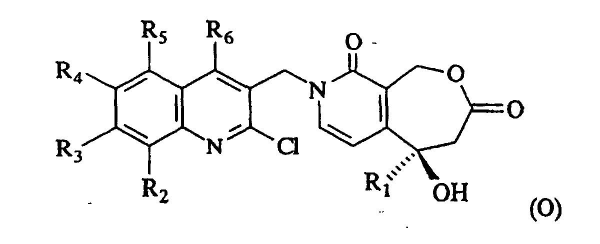 dans laquelle R₂, R₃, R₄, R₅ et R₆ ont la signification indiquée ci-dessus.
- le composé (O) est ensuite cyclisé pour donner le composé de formule (I).

La formation des composés (O) à partir des composés de formules générales (M) et (N) s'effectue par un traitement connu de l'homme de l'art sous le nom de réaction de Mitsunobu (se référer à Mitsunobu, O. et coll. *Synthesis,* p. 1 (1981)). Il s'agit de déplacer la fonction hydroxyle du composé (N) par un nucléophile tel que le composé (M), ou un dérivé déprotoné de ce dernier, par un traitement avec une phosphine, par exemple la triphénylphosphine, et un dérivé azodicarboxylate, par exemple l'azodicarboxylate de diéthyle ou de diisopropyle, dans un solvant aprotique tel que, par exemple, le tétrahydrofurane ou le *N,N-*diméthylformamide. La cyclisation des composés (O) pour donner les composés de formule (I) s'effectue de préférence en présence d'un catalyseur palladié (par exemple le diacétate de palladium) dans des conditions basiques (fournies par exemple par un acétate alcalin éventuellement combiné à un agent de transfert de phase tel que par exemple le bromure de tétrabutylammonium), dans un solvant aprotique tel que l'acétonitrile ou le *N,N*-diméthylformamide, à une température comprise entre 50° C et 120° C (R. Grigg et coll., *Tetrahedron* 46, p. 4003 (1990)).

Le composé de formule (M) est préparé selon un procédé constitué des étapes successives suivantes :
- un ester racémique représenté ci-dessous 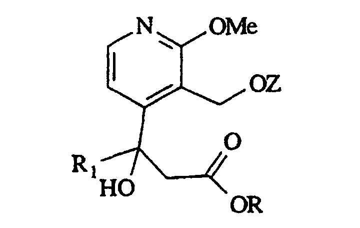 dans lequel R₁ a la signification indiquée ci-dessus, R est un alkyle inférieur et Z un groupement protecteur de la fonction alcool (pour sa préparation, se référer notamment à la demande de brevet WO 97/00876) est transformé en l'acide carboxylique correspondant ;
- on soumet ensuite ce composé à une opération de séparation des énantiomères, connue de l'homme de l'art sous le nom de résolution (cf Jacques, et coll., *"Enantiomers, Racemates and Resolution",* 2nd édition, Wiley, New-York, **1991**), et -permettant l'obtention du composé enantiomériquement enrichi de formule générale (A) 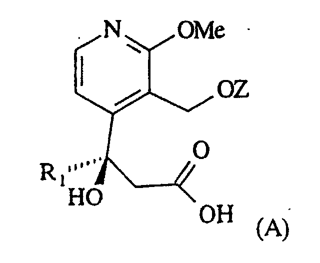 dans laquelle R₁ et Z ont la signification indiquée ci-dessus ;
- la fonction alcool du composé de formule générale (A) est ensuite déprotégée pour donner le produit de formule générale (B) 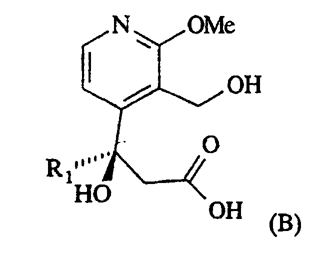 dans laquelle R₁ a la signification indiquée ci-dessus,
- le composé de formule générale (B) est ensuite cyclisé pour obtenir le composé de formule générale (C) 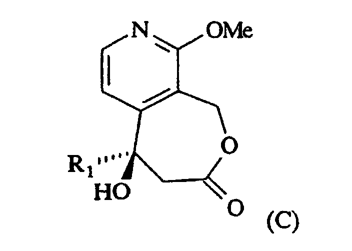 dans laquelle R₁ a la signification indiquée ci-dessus,
- enfin, le groupement méthoxy du composé de formule générale (C) est transformé en carbonyle pour obtenir un composé de formule générale (M) 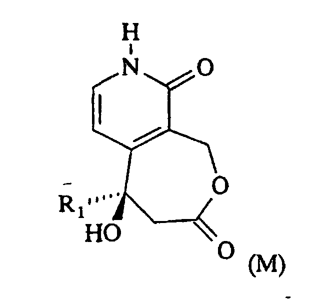 dans laquelle R₁ a la signification indiquée ci-dessus.

Dans le cas particulier où R₁ représente un groupe éthyle, R représente un *tert*-butyl et Z représente un groupe benzyle, le composé de formule (M) est synthétisé selon le procédé constitué des étapes successives suivantes :
- l'ester *t*-butylique racémique représenté ci-dessous (pour sa préparation, se référer notamment à la demande de brevet WO 97/00876) 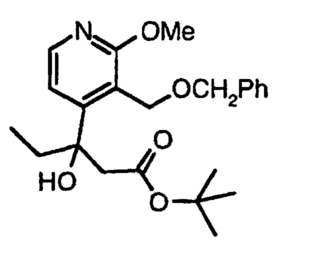 est traité par de l'acide trifluoroacétique durant 18 h à température ambiante pour donner l'acide carboxylique correspondant ;
- on chauffe dans de l'alcool isopropylique le sel de quinidine de l'acide 3-(3-benzyloxyméthyl-2-méthoxy-4-pyridyl)-3-hydroxy-pentanoïque à une température supérieure à 30° C, et de préférence de 50° C environ, avant de laisser refroidir le milieu réactionnel jusqu'à température ambiante, de sorte que le sel de l'énantiomère (+) de l'acide 3-(3-benzyloxyméthyl-2-méthoxy-4-pyridyl)-3-hydroxy-pentanoïque cristallise tandis que le sel de l'isomère (-), dont l'anion est représenté ci-dessous, reste en solution 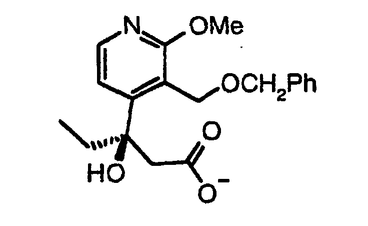
- la solution dans l'alcool isopropylique du sel de l'énantiomère (-) de l'acide 3-(3-benzyloxyméthyl-2-méthoxy-4-pyridyl)-3-hydroxy-pentanoïque est concentrée et traitée à l'acide chlorhydrique pour donner le composé de formule (A') 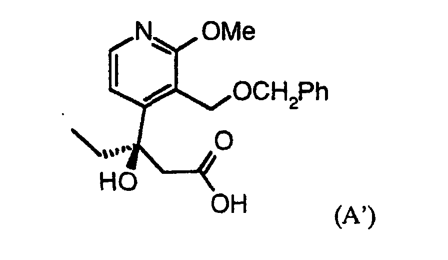
- le composé (A') est ensuite mis en contact avec du palladium en présence d'une source d'hydrogène pour donner le produit débenzylé de formule (B') 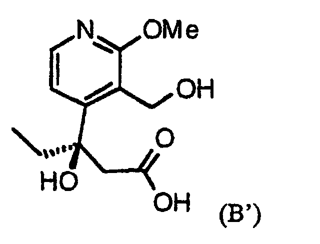
- on cyclise ensuite le composé de formule (B') pour obtenir le composé de formule (C') 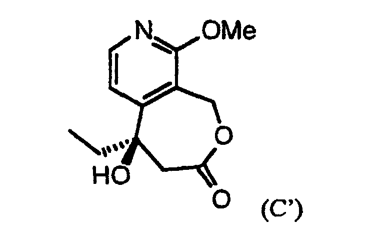
- enfin, on transforme le groupement méthoxy du composé de formule (C') en carbonyle, pour obtenir la (+)-5-éthyl-5-hydroxy-1,3,4,5,8,9-hexahydrooxépino[3,4-c]pyridin-3,9-dione (ou (+)-EHHOPD) représentée ci-dessous.

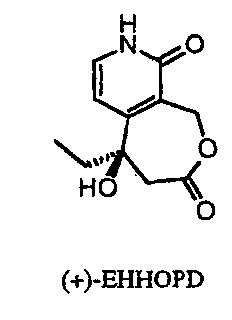

Pour le procédé décrit ci-dessus, la réaction conduisant du composé de formule (A') au composé de formule (B') se ferra de préférence dans du méthanol, et de préférence en chauffant le milieu réactionnel à 40° C environ après l'addition de formiate d'ammonium. La cyclisation du composé de formule (B') pour donner le composé (C') pourra s'effectuer dans du THF, de préférence à une température de 50° C environ. Pour la réaction conduisant du composé de formule (C') au (+)-EHHOPD, on travaillera de préférence à température ambiante avec l'acétonitrile comme solvant.

Les composés de formule (I_{A}) dans laquelle au moins un des radicaux R₂, R₃, R₄ et R₅ représente un sulfonate (comme par exemple mésylate, triflate ou tosylate), peuvent être obtenus par un procédé caractérisé en ce que l'on traite le composé hydroxylé correspondant dans un solvant aprotique anhydre avec un agent sulfonylant en présence d'une base. Le solvant aprotique peut être le dichlorométhane ou le N,N-diméthylformamide, l'agent sulfonylant le chlorure de méthanesulfonyle, l'anhydride triflique, le N-phényltriflimide ou le chlorure de p-toluène sulfonyle, et la base la triéthylamine, la pyridine ou l'hydrure de sodium. Le groupement sulfonate peut également être introduit au niveau des produits intermédiaires.

Les composés de formule (N), dans laquelle R₆ est un atome d'hydrogène et R₂, R₃, R₄ et R₅ ont la signification indiquée ci-dessus, peuvent être obtenus à partir d'anilines de formule (P) 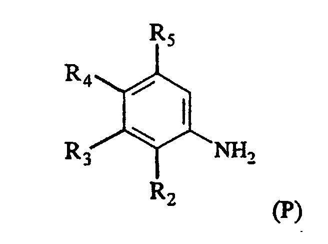 dans laquelle R₂, R₃, R₄ et R₅ ont la signification indiquée ci-dessus, selon le procédé suivant : une aniline de formule (P) est *N*-acétylée par traitement avec un agent acétylant tel que par exemple l'anhydride acétique. L'acétanilide ainsi obtenu est traité à une température comprise entre 50° C et 100° C, de préférence environ 75° C, par un réactif connu de l'homme de l'art sous le nom de réactif de Vilsmeyer (obtenu par l'action de l'oxychlorure de phosphoryle sur la *N,N*-diméthylformamide à une température comprise entre 0° C et 10° C) pour donner le 2-chloro-3-quinoléinecarbaldéhyde correspondant (se référer, par exemple à Meth-Cohn, et coll. *J. Chem. Soc., Perkin Trans. I p.* 1520 (1981) ; Meth-Cohn, et coll *J. Chem. Soc., Perkin Trans. I* p. 2509 (1981); et Nakasimhan et coll. *J. Am. Chem. Soc., 112,* p. 4431 (1990)). Cet intermédiaire est facilement réduit en le quinolylméthanol correspondant de formule (N), dans des conditions classiques connues de l'homme de l'art telles que le traitement dans un solvant alcoolique (par exemple le méthanol) par du borohydrure de sodium à une température comprise entre 0° C et 40° C.

Les composés de formule (N) dans laquelle R₂, R₃, R₄, R₅ et R₆ ont la signification indiquée ci-dessus, peuvent aussi être obtenus à partir de quinolones carboxylées de formule (Q) 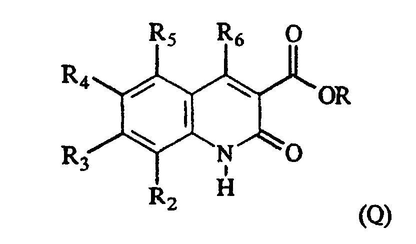 dans laquelle R₂, R₃, R₄, R₅ et R₆ ont la signification indiquée ci-dessus, selon le procédé suivant: une quinolone de formule (Q) est chlorée pour donner la chloroquinoléine correspondante dont la fonction carboxylée est réduite pour donner le composé de formule générale (N). La chloration peut être effectuée avec un oxyde de chlorophosphine tel que l'oxychlorure de phosphore ou l'oxyde de chlorodiphénylphosphine, purs ou en présence d'un cosolvant aprotique inerte tel que le toluène ou le chloroforme, à une température comprise entre 50° C et 120° C. La chloration s'effectue de préférence avec un excès d'oxychlorure de phosphore à 80° C. La réduction peut être effectuée avec un hydrure d'aluminium dans un solvant aprotique tel que l'éther diéthylique, l'oxyde de *tert*-butylméthyle, le tétrahydrofurane, le dichlorométhane, le chloroforme, le trichloroéthane ou le toluène, à une température comprise entre 0° C et 50° C. La réduction s'effectue de préférence avec de l'hydrure de diisobutylaluminium dans le dichlorométhane à température ambiante.

Les composés de formule (Q), dans laquelle et R₆ est un atome d'hydrogène et R₂, R₃, R₄ et R₅ ont la signification indiquée ci-dessus, peuvent être obtenus à partir d'acides anthraniliques de formule (R) 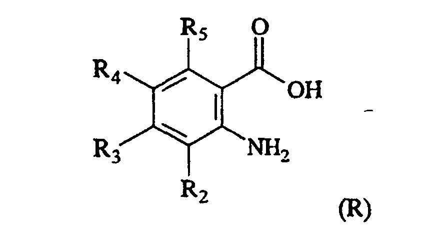 dans laquelle R₂, R₃, R₄ et R₅ ont la signification indiquée ci-dessus, selon le procédé suivant: un acide de formule (R) est réduit pour donner l'alcool benzylique correspondant. La fonction alcoolique de l'intermédiaire ainsi obtenu est protégée sélectivement afin de laisser la fonction amine intacte. L'aniline résultante est acylée avec un dérivé de l'acide malonique. La fonction alcoolique précédemment protégée est déprotégée, puis oxydée pour donner la fonction carbonyle correspondante, et l'intermédiaire ainsi obtenu est soumis à un processus intramoléculaire, selon une réaction connue de l'homme de l'art sous le nom de condensation de Knovenagel, pour donner des quinolones carboxylées de formule (Q), dans laquelle R₆ est un atome d'hydrogène et R₂, R₃, R₄ et R₅ ont la signification indiquée ci-dessus. La réduction de l'acide en alcool peut être effectuée par un hydrure métallique dans un solvant aprotique inerte à une température comprise entre 0° C et 50° C, et de préférence par l'hydrure mixte de lithium et d'aluminium dans le tétrahydrofurane à température ambiante. La protection de l'alcool benzylique intermédiaire peut être effectuée selon des méthodes générales connues de l'homme de l'art (Greene T, et coll., *"Protective groups in Organic Synthesis",* 2nd édition, Wiley, New-York, 1991) ou encore avec une chlorure de silyle en présence d'une base, dans un solvant aprotique à une température comprise entre 0° C et 50° C, et de préférence par le chlorure de *tert*-butyldiphénylsilyle en présence d'imidazole, dans le diméthylformamide à température ambiante. L'acylation peut être effectuée avec un dérivé malonique tel que le chlorure d'éthylmalonyle ou le malonate de méthyle, en présence d'une base telle que la triéthylamine ou la 4-diméthylaminopyridine, dans un solvant aprotique tel que l'acétonitrile, le tétrahydrofurane ou le toluène, à une température comprise entre 0° C et 110° C, et de préférence avec du chlorure d'éthylmalonyle dans l'acétonitrile à température ambiante, en présence de triéthylamine. La déprotection peut être effectuée selon le groupe protecteur de l'alcool benzylique précédemment choisi (Greene, T.), et dans le cas d'éther silylés par une source d'ion fluorure telle que le fluorure de césium, ou de potassium en présence d'un agent de transfert de phase, ou encore le fluorure de tétrabutylammonium, dans un solvant aprotique tel que le tétrahydrofurane, à une température comprise entre 0° C et 50° C, et de préférence à température ambiante. L'oxydation peut être effectué en présence de sels de chrome (VI) portant des ligands pyridyle, par le réactif de Swern, ou encore par le complexe pyridine-trioxyde de soufre dans le diméthyl sulfoxyde en présence de triéthylamine, et de préférence par le dichromate de pyridinium dans le dichlorométhane à température ambiante. La condensation de Knoevenagel intramoléculaire peut s'effectuer spontanément ou en solution en présence d'une base, et de préférence dans le dichlorométhane en présence de triéthylamine à température ambiante.

Les composés de formule (Q) dans laquelle R₂, R₃, R₄, R₅ et R₆ ont la signification indiquée ci-dessus, peuvent être obtenus à partir d'aminocétones de formule (S) 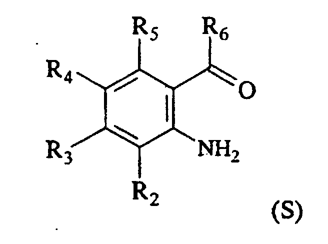 dans laquelle R₂, R₃, R₄, R₅ et R₆ ont la signification indiquée ci-dessus, selon le procédé suivant : une aminocétone (S) est acylée avec un dérivé de l'acide malonique et l'intermédiaire ainsi obtenu est soumis à un processus intramoléculaire, selon une réaction connue de l'homme de l'art sous le nom de condensation de Knovenagel, pour donner des quinolones carboxylées de formule (Q). L'acylation peut être effectuée avec un dérivé malonique tel que le chlorure d'éthylmalonyle ou le malonate de méthyle, en présence d'une base telle que la triéthylamine ou la 4-diméthylamino-pyridine, dans un solvant aprotique tel que l'acétonitrile, le tétrahydrofurane ou le toluène, à une température comprise entre 0° C et 110° C, et de préférence avec du chlorure d'éthylmalonyle dans l'acétonitrile à température ambiante, en présence de triéthylamine. La condensation de Knoevenagel intramoléculaire peut s'effectuer spontanément ou en solution en présence d'une base, et de préférence dans l'acétonitrile en présence d'éthylate de sodium à température ambiante.

Les aminocétones de formule (S), dans laquelle R₂, R₃, R₄, R₅ et R₆ ont la signification indiquée ci-dessus, peuvent être obtenus à partir de benzonitriles orto-aminés de formule 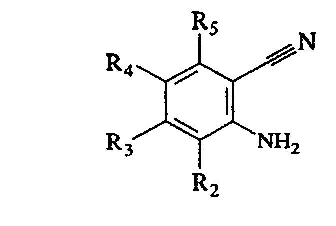 dans laquelle R₂, R₃, R₄ et R₅ ont la signification indiquée ci-dessus, par traitement par avec un réactif de Grignard de formule R₆-MgX, où X est un halogène et R₆ a la signification ci-dessus, selon des méthodes connues de l'homme de l'art.

Les aminocétones de formule (S), dans laquelle R₆ est un radical aryle et R₂, R₃, R₄ et R₅ ont la signification indiquée ci-dessus, peuvent être obtenus à partir d'acides anthraniliques de formule (R) décrite ci-dessus, par traitement par du chlorure de benzoyle au reflux pour donner une benzoxazone qui peut être transformée en présence d'un réactif de Grignard de formule R₆-MgX, où X est un halogène et R₆ est un radical aryle en la benzophénone ortho-aminée correspondante, laquelle peut être débenzoylée par des réactifs tels que, par exemple, le bromure d'hydrogène en solution dans l'eau ou dans l'acide acétique glacial.

Les aminocétones de formule (S) dans laquelle R₂, R₃, R₄, R₅ et R₆ ont la signification indiquée ci-dessus, peuvent être obtenus à partir d'anilines de formule (P) dans laquelle R₂, R₃, R₄ et R₅ ont la signification indiquée ci-dessus, selon le procédé suivant : l'atome d'azote d'une aniline de formule (P) est acylée avec un agent conférant un caractère ortho-directeur dans la réaction de métallation des aryles, et le composé ainsi obtenu est métallé, puis traité par un aldéhyde de formule R₆-CHO dans laquelle R₆ a la signification ci-dessus. Le procédé est alors complété par une oxydation de l'intermédiaire alcoolique ainsi obtenue puis par une libération de la fonction azotée pour donner une aminocétone de formule (S). Pour ce procédé, le passage à la fonction ortho-directrice peut être obtenue par traitement d'une aniline (P) avec un agent "bocant", et de préférence par le dicarbonate de di*-tert* butyle dans un solvant aprotique tel que le tétrahydrofurane, le dioxane ou le diméthoxyéthane à la température de reflux. La métallation peut être obtenue par traitement avec un réactif lithié tel que le tert-butyllithium, le *sec*-butyllithium, le mésityllithium, ou, en présence de tétraméthyl-éthylènediamine, le n-butyllithium, et de préférence le n-butyllithium en présence de tétraméthyl-éthylènediamine, dans un solvant aprotique tel que tétrahydrofurane, le dioxane ou le diméthoxyéthane, à une température comprise entre -80° C et 0° C. L'oxydation peut être effectuée en présence de sels de chrome (VI) portant des ligands pyridyle, par le réactif de Swern, ou encore par le complexe pyridine-trioxyde de soufre dans le diméthylsulfoxyde en présence de triéthylamine, et de préférence par le dichromate de pyridinium dans le dichlorométhane au reflux. La fonction azotée peut être obtenue par un traitement en milieu acide, et de préférence par l'acide trifluoroacétique dans le dichlorométhane à température ambiante.

Des analogues des composés intermédiaires du type de (N) ont été décrits précédemment et en particulier dans la demande WO 95/05427.

Les composés de Formule (I_{A}) peuvent aussi être obtenus par un procédé nouveau, caractérisé en ce que l'on traite un composé de formule (IV) 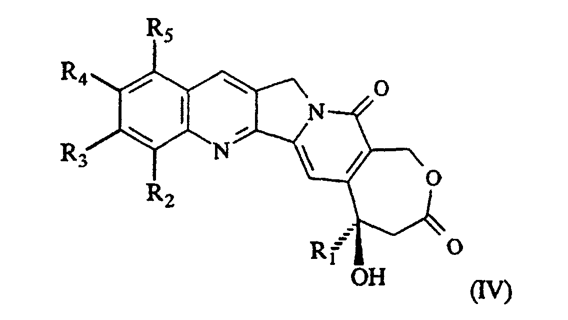 dans laquelle R₁, R₂, R₃, R₄ et R₅ ont la signification indiquée ci-dessus, dans un milieu fortement acide, en présence d'un sel de fer (III) et d'un précurseur du radical libre R₆, par une solution contenant des radicaux hydroxyde ou alkoxyde.

Bien que l'art antérieur fasse mention de l'application d'une réaction similaire aux analogues de la camptothécine contenant une α-hydroxylactone (Sawada, S., et coll., *Chem Pharm. Bull.,* (1991), vol. 39, p. 2574) ; demande PCT WO 98/35940), son utilisation pour les analogues de la camptothécine tels que les composés de formule (IV), contenant une β-hydroxylactone, n'a pas été prévue et est inattendue, car en milieu fortement acide une fonction hydroxyle ternaire et benzylique, en position β par rapport à une fonction carboxylique, est généralement éliminée pour donner l'oléfine correspondante (Nagasawa, et coll. *Heterocycles* 1989, vol. 28, p. 703 ; Kimura, H. et coll., *Chem. Pharm. Bull.* 1982, vol. 30, p. 552 ; Fujita, T. et coll., *J. Appl Chem Biotechnol.* 1982, vol. 32, p. 421 ; Miller, R. E., et coll., *J. Org. Chem.* 1950, vol. 15, p. 89 ; Fieser, L. F., et coll., J. Am. Chem. Soc. 1948, vol. 70, p. 3209).

Dans le procédé ci-dessus, le milieu fortement acide peut être fourni par des acides tels que l'acide trifluoroacétique ou l'acide sulfurique, aqueux ou non-aqueux, et de préférence l'acide sulfurique aqueux ; le sel de fer (III) sera de préférence le sulfate de fer (III) heptahydraté, et le précurseur de radical libre un aldéhyde de formule R₆-CHO. La solution contenant des radicaux hydroxydes ou alkoxydes peut être fournie par de l'eau oxygénée ou de l'hydroperoxyde de *tert*-butyle, et de préférence par de l'eau oxygénée à 30 volumes.

Les composés de la présente invention possèdent d'intéressantes propriétés pharmacologiques. C'est ainsi que les composés de la présente invention ont une activité inhibitrice de la topoisomérase I et/ou II et une activité anti-tumorale. L'état de la technique suggère que les composés de l'invention présentent une activité anti-parasitaire et/ou anti-virale. Les composés de la présente invention peuvent ainsi être utilisés dans différentes applications thérapeutiques.

On trouvera ci-après, dans la partie expérimentale, une illustration des propriétés pharmacologiques des composés de l'invention.

Les composés peuvent inhiber la topoisomérase, par exemple de type I et/ou II, chez un patient, par exemple un mammifère tel que l'homme, par administration à ce patient d'une quantité thérapeutiquement efficace d'un des composés de l'invention.

Les composés de l'invention possèdent une activité anti-tumorale. Ils peuvent être utilisés pour le traitement des tumeurs, par exemple des tumeurs exprimant une topoisomérase, chez un patient par administration audit patient d'une quantité thérapeutiquement efficace d'un des composés de l'invention. Des exemples de tumeurs ou de cancers comprennent les cancers de l'oesophage, de l'estomac, des intestins, du rectum, de la cavité orale, du pharynx, du larynx, du poumon, du colon, du sein, du cervix uteri, du corpus endométrium, des ovaires, de la prostate, des testicules, de la vessie, des reins, du foie, du pancréas, des os, des tissus conjonctifs, de la peau, par exemple les mélanomes, des yeux, du cerveau et du système nerveux central, ainsi que le cancer de la thyroïde, la leucémie, la maladie de Hodgkin, les lymphomes autres que ceux de Hodgkin, les myélomes multiples et autres.

Ils peuvent également être utilisés pour le traitement des infections parasitiques par l'inhibition des hémoflagellates (par exemple dans la trypanosomie ou les infections leishmania) ou par inhibition de la plasmodie (comme par exemple dans la malaria), mais aussi le traitement des infections ou maladies virales.

Ces propriétés rendent les composés de l'invention aptes à une utilisation pharmaceutique. La présente demande a également pour objet, à titre de médicaments, les composés de l'invention de formule générale (I_{A}) telle que définie ci-dessus. L'invention concerne de même les compositions pharmaceutiques contenant, à titre de principe actif, l'un au moins des médicaments tels que définis ci-dessus.

L'invention concerne ainsi des compositions pharmaceutiques contenant un composé de l'invention ou un sel additif d'acide pharmaceutiquement acceptable de celui-ci, en association avec un support pharmaceutiquement acceptable suivant le mode d'administration choisie (par exemple orale, intraveineuse, intrapéritonéales, intramusculaires, trans-dermique ou sous-cutanée). La composition pharmaceutique (par exemple thérapeutique) peut être sous forme solide, liquide, de liposomes ou de micelles lipidiques.

La composition pharmaceutique peut être sous forme solide comme, par exemple, les poudres, pilules, granules, comprimés, liposomes, gélules ou suppositoires. La pilule, le comprimé ou la gélule peuvent être revêtus d'une substance capable de protéger la composition de l'action de l'acide gastrique ou des enzymes dans l'estomac du sujet pendant une période de temps suffisante pour permettre à cette composition de passer non digérée dans l'intestin grêle de ce dernier. Le composé peut aussi être administré localement, par exemple à l'emplacement même de la tumeur. Le composé peut aussi être administré selon le processus de la libération prolongée (par exemple une composition à libération prolongée ou une pompe d'infusion). Les supports solides appropriés peuvent être, par exemple, le phosphate de calcium, le stéarate de magnésium, le carbonate de magnésium, le talc, les sucres, le lactose, la dextrine, l'amidon, la gélatine, la cellulose, la cellulose de méthyle, la cellulose carboxyméthyle de sodium, la polyvinylpyrrolidine et la cire. Les compositions pharmaceutiques contenant un composé de l'invention peuvent donc également se présenter sous forme liquide comme, par exemple, des solutions, des émulsions, des suspensions ou une formulation à libération prolongée. Les supports liquides appropriés peuvent être, par exemple, l'eau, les solvants organiques tels que le glycérol ou les glycols tel que le polyéthylène glycol, de même que leurs mélanges, dans des proportions variées, dans l'eau.

L'invention a également pour objet l'utilisation des composés de l'invention pour la préparation de médicaments destinés à inhiber les topoisomérases, et plus particulièrement les topoisomérases de type I ou de type II, de médicaments destinés à traiter les tumeurs, de médicaments destinés à traiter les infections parasitaires, ainsi que de médicaments destinés à traiter des infections ou maladies virales.

La dose d'un composé selon la présente invention, à prévoir pour le traitement des maladies ou troubles mentionnés ci-dessus, varie suivant le mode d'administration, l'âge et le poids corporel du sujet à traiter ainsi que l'état de ce dernier, et il en sera décidé en définitive par le médecin ou le vétérinaire traitant Une telle quantité déterminée par le médecin ou le vétérinaire traitant est appelée ici "quantité thérapeutiquement efficace".

A moins qu'ils ne soient définis d'une autre manière, tous les termes techniques et scientifiques utilisés ici ont la même signification que celle couramment comprise par un spécialiste ordinaire du domaine auquel appartient cette invention.

Les exemples suivants sont présentés pour illustrer les procédures ci-dessus.

### PARTIE EXPERIMENTALE :

### Exemple 1 : (5R)-5-éthyl-11-fluoro-5-hydroxy-4,5,13,15-tétrahydro-1H,3H-oxépino[3',4':6,7]indolizino[1,2-b]quinoléine-3,15-dione (ne fait pas partie de l'invention)

### Etape 1a. : (3R)- 3-(3-benzyloxyméthyl-2-méthoxy-4-pyridyl)-3-hydroxy-pentanoate de quinidinium

Du 3-(3-benzyloxyméthyl-2-méthoxy-4-pyridyl)-3-hydroxy-pentanoate de *tert*-butyle (obtenu selon la méthode décrite dans la demande de brevet PCT WO 97/00876 ; 40 g ; 100 mmol) est traité par de l'acide trifluoroacétique (150 ml) et le milieu réactionnel est agité pendant 18 h à 20° C, puis concentré sous pression réduite. Le résidu, repris par une solution aqueuse saturée en bicarbonate de sodium (200 ml), est lavé au dichlorométhane (2 x 100 ml) et la solution résultante est acidifiée à pH = 1 avec de l'acide chlorhydrique 6 N, puis extraite au dichlorométhane (2 x 200 ml). Les extraits combinés sont séchés sur sulfate de magnésium et concentrés. La solution est séchée sur du sulfate de magnésium et concentrée. L'acide racémique ainsi obtenu (31,1 g ; 90 mmol), repris dans l'alcool isopropylique (30 ml), est traité par une solution de quinidine (29,2 g; 90 mmol) dans l'alcool isopropylique (30 ml), et le mélange résultant est agité à 50° C jusqu'à dissolution complète. On laisse alors la température redescendre à 40° C, l'agitation est arrêtée et on laisse refroidir à température ambiante. Le milieu est amené à 0° C sans agitation puis maintenu à cette température pendant 16 h. On laisse ensuite la température remonter jusqu'à 20° C et on agite jusqu'à cristallisation. Le milieu est dilué par de l'alcool isopropylique puis filtré. Le précipité est rincé par de l'alcool isopropylique. Le sel dextrogyre précipite alors que le sel lévogyre reste en solution dans l'alcool isopropylique. On recueille le filtrat qui est concentré pour donner le produit attendu. Une analyse par HPLC (colonne CHIRAL-AGP 5µ (10 cm x 4 mm) éluée par un mélange alcool isopropylique/eau/tampon phosphate pH 6,5 30/920/50, à un débit de 1,2 ml/min, détection UV à 280 nm) révèle des temps de rétention de 6,4 min pour le sel lévogyre et de 2,8 min pour le sel dextrogyre et un rapport diastéréoisomérique de 83/17.

### Etape 1b : (SR)-5-éthyl-5-hydroxy-1,3,4,5,8,9-hexahydrooxépino[3,4-c]pyridin-3,9-dione, ou (+)-EHHOPD

Le résidu obtenu à l'étape la est agité pendant 16h à 20° C dans un mélange de dichlorométhane (270 ml) et d'acide chlorhydrique 1N (270 ml). Après décantation, la phase organique est concentrée, et le résidu est repris dans du méthanol (87 ml) pour être engagé dans la phase suivante. Cette solution est coulée sous azote sur du Palladium à 10 % sur charbon humide à 50 % (27,7 g ; 13 mmol). Le milieu réactionnel est agité pendant 5 min, puis est coulée une solution de formiate d'ammonium (11,5 g ; 183 mmol) dans du méthanol (135 ml). Le milieu réactionnel est agité pendant 30 min en laissant la température évoluer, puis il est chauffé à 40° C pendant 30 min. Le milieu est alors filtré sur lit de Clarcel et concentré. On coule du toluène (40 ml) que l'on évapore, et cette opération est répétée afin d'éliminer les traces de méthanol. Le résidu, repris dans du tétrahydrofurane (45 ml), est traité par une solution de dicydohexylcarbodiimide (7,18g ; 34,5 mmol) dans du tétrahydrofurane (20ml). Le milieu réactionnel est chauffé à 50° C pendant 1 h, puis ramené à 20° C, et la dicyclohexylurée est filtrée. Le filtrat est concentré à sec et le résidu, repris dans de l'acétonitrile (46 ml), est traité par de l'iodure de sodium (6,0 g ; 40,5 mmol) et du chlorure de triméthylsilyle (5,13 ml ; 40,5 mmol). Le milieu réactionnel est maintenu sous agitation à température ambiante pendant 5 h, puis additionné d'acétonitrile (28 ml) et d'eau (5,6 ml). Le précipité obtenu est recueilli par filtration, puis repris dans de l'eau (10 ml), et le mélange obtenu est neutralisé à l'aide d'une solution d'ammoniaque. Le précipité est recueilli par filtration puis repris dans de l'acétone (40 ml) à laquelle on ajoute de l'eau (150 ml). Les cristaux formés sont recueillis par filtration et séchés pour donner 3 g de (+)-EHHOPD avec une proportion énantiomérique de 99,4/0,6.
RMN ¹H (DMSO-d6, δ) : 0,8 (t, 3H) ; 1,65 (m, 2H) ; 3,00-3,35 (q, 2H) ; 5,3 (q, 2H) ; 5,7 (s, 1H) ; 6,35 (d, 1H) ; 7,3 (d, 1H) ; 11,7 (s, 1H).

### Etape 1c : 2-amino-6-fluorophénylméthanol

Une solution sous argon d'acide 2-amino-6-fluorobenzoïque (5 g ; 32 mmol) dans du tétrahydrofurane anhydre (100 ml) est traitée à température ambiante par de l'hydrure de lithiumaluminium (1M dans le tétrahydrofurane; 64 ml ; 64 mmol). Le milieu réactionnel est agité pendant 3 h, puis hydrolysé à 0° C par une solution aqueuse saturée en chlorure d'ammonium (100 ml). Le mélange résultant est extrait à l'acétate d'éthyle (2 x 70 ml). Les extraits combinés sont lavés à l'eau et avec une solution aqueuse saturée en chlorure de sodium, puis séchés sur sulfate de magnésium et concentrés pour donner 3,8 g du produit désiré, un solide blanc (P_{f} : 93° C).
IR (KBr) : 784, 1001, 1471, 1591, 1621 cm⁻¹.
RMN ¹H (DMSO-d6, δ) : 4,44 (dd, 2H) ; 4,93 (t, 1H) ; 5,27 (s, 2H) ; 6,27 (t, 1H) ; 6,45 (d, 1H) ; 6,96 (q, 1H).

### Etape 1d: 2-(3-fluoro-2-hydroxyméthylphénylcarbamoyl)acétate d'éthyle

Une solution d'aminobenzylalcool (obtenue à l'étape 1c ; 3,8 g ; 27 mmol) et d'imidazole (4,3 g ; 64 mmol) dans du *N,N*-diméthylformamide (52 ml) est traitée par du chlorure de tert-butyldiphénylsilyle (8,37 ml ; 32 mmol). Le mélange résultant est agité pendant 2 h à température ambiante, puis est additionné d'eau (100 ml) et extrait à l'acétate d'éthyle (2 x 60 ml). Les extraits combinés sont lavés à l'eau et avec une solution aqueuse saturée en chlorure de sodium, puis séchés sur sulfate de magnésium et concentrés. L'intermédiaire silylé ainsi obtenu (10 g) est repris dans l'acétonitrile (52 ml), puis de la triéthylamine (4,5 ml ; 32,4 mmol) est ajoutée à la solution, et le mélange résultant est traité au goutte à goutte par du chlorure d'éthylmalonyle (4,15 ml ; 32,4 mmol). Le mélange résultant est agité pendant 2 h à température ambiante, puis additionné d'eau (100 ml) et extrait à l'acétate d'éthyle (2 x 60 ml). Les extraits combinés sont lavés à l'eau et avec une solution aqueuse saturée en chlorure de sodium, puis séchés sur sulfate de magnésium et concentrés. Le résidu (16 g) est repris au tétrahydrofurane (50 ml) et traité au goutte à goutte par du fluorure de tétrabutylammonium (1M dans le tétrahydrofurane ; 27 ml ; 27 mmol). Le mélange résultant est agité pendant 1 h à température ambiante, puis est additionné d'eau (100 ml) et extrait à l'acétate d'éthyle (2 x 60 ml). Les extraits combinés sont lavés à l'eau et avec une solution aqueuse saturée en chlorure de sodium, puis séchés sur sulfate de magnésium et concentrés. La purification du résidu par chromatographie à moyenne pression (SiO₂, CH₂Cl₂/MeOH, 95/5) donne 4,8 g d'un solide blanc (P_{f} : 91° C).
IR (KBr) : 1472, 1542, 1589, 1657, 1719, 3286, 3482 cm⁻¹.
RMN ¹H (DMSO-d6, δ) : 1,19 (t, 3H) ; 3,54 (s, 2H) ; 4,14 (q, 2H) ; 4,55 (dd, 2H) ; 5,21 (t, 1H) ; 6,97 (t, 1H) ; 7,31 (dd, 1H) ; 7,53 (d, 1H).

### Etape 1e : 5-fluoro-2-oxo-1,2-dihydro-3-quinoléinecarboxylate d'éthyle

Une solution de dérivé malonique (obtenu à l'étape 1d ; 4,8 g ; 19 mmol) dans du dichlorométhane (280ml) est traitée par du dichromate de pyridinium (8,3 g ; 22 mmol). La suspension résultant est agitée pendant 4 h à température ambiante, puis traitée avec de la triéthylamine (30 ml ; 220 mmol). Le milieu réactionnel est agité à température ambiante pendant 16 h, puis concentré sous pression réduite. La purification du résidu par chromatographie à moyenne pression (SiO₂, CH₂Cl₂/MeOH, 95/5) donne 2,1 g d'un solide jaune (P_{f} : 180° C).
IR (KBr) : 1441, 1498, 1655, 1747 cm⁻¹.
RMN ¹H (DMSO-d6, δ) : 1,31 (t, 3H) ; 4,28 (q, 2H) ; 7,06 (t, 1H) ; 7,16 (d, 1H) ; 7,61 (dd, 1H) ; 8,43 (s, 1H); 12,27 (s, 1H).

### Etape 1f: 2-chloro-5-fluoro-3-quinoléinecarboxylate d'éthyle

La quinolone (obtenue à l'étape 1e ; 2,1 g) est chauffée à 80° C dans de l'oxychlorure de phosphore (14 ml) jusqu'à réaction complète (contrôle CCM : SiO₂, CH₂Cl₂/MeOH, 95/5). La solution résultante est alors concentrée sous pression réduite et le résidu est repris à l'eau. Le précipité ainsi formé est recueilli par filtration, lavé à l'eau jusqu'à pH neutre, et séché sous pression réduite en présence de pentoxyde de phosphore pour donner 1,8 g d'un solide blanc (P_{f} : 97° C).
IR (KBr) : 1268, 1631, 1723 cm⁻¹.
RMN ¹H (DMSO-d6, δ) : 1,38 (t, 3H) ; 4,42 (q, 2H) ; 7,60 (t, 1H) ; 7,89 (d, 1H) ; 7,97 (dd, 1H) ; 8,92 (s, 1H).

### Etape 1g : 2-chloro-5-fluoro-3-quinolylméthanol

Une solution de quinoléinecarboxylate (obtenu à l'étape 1f ; 1,8 g ; 6,7 mmol) dans du dichlorométhane (40 ml) sous argon est traitée au goutte à goutte par de l'hydrure de diisobutylaluminium (1M dans le dichlorométhane ; 20 ml ; 20 mmol) à une température maintenue à 10° C par un bain d'eau glacée. Le mélange réactionnel est agité pendant 1 h à température ambiante, puis versé sur une solution de tartrate de sodium et de potassium à 20 % (200 ml). Le mélange résultant est agité vigoureusement pendant 1 h, puis filtré sur célite. Le filtrat est extrait au dichlorométhane (2 x 100 ml). Les extraits combinés sont lavés à l'eau et avec une solution aqueuse saturée en chlorure de sodium, puis séchés sur sulfate de magnésium et concentrés. La purification du résidu par chromatographie à moyenne pression (SiO₂, CH₂Cl₂/MeOH, 98/2) donne 450 mg d'un solide blanc (P_{f} : 176° C).
RMN ¹H (DMSO-d6, δ) : 4,71 (d, 2H) ; 5,78 (t, 3H) ; 7,51 (t, 1H) ; 7,75-7,83 (m, 2H) ; 8,50 (s, 1H).

### Etape 1h : (5R)-5-éthyl-11-fluoro-5-hydroxy-4,5,13,15-tétrahydro-1H,3H-oxépino[3',4':6,7]indolizino[1,2-b]quinoléine-3,15-dione

Une solution de quinolylméthanol (obtenu à l'étape 1g ; 422 mg ; 2 mmol), de (+)-EHHOPD (obtenu à l'étape 1b ; 446 mg ; 2 mmol) et de triphénylphosphine (592 mg ; 2,2 mmol) dans du *N,N*-diméthylformamide (8 ml) est traitée au goutte à goutte par de l'azodicarboxylate d'isopropyle (0,43 ml ; 2,2 mmol). Le mélange réactionnel est agité pendant 16 h à température ambiante, puis additionné d'eau (100 ml) et extrait à l'acétate d'éthyle (2 x 100 ml). Les extraits combinés sont lavés à l'eau et avec une solution aqueuse saturée en chlorure de sodium, puis séchés sur sulfate de magnésium et concentrés sous pression réduite. Le résidu est purifié par chromatographie à moyenne pression (SiO₂, AcOEt/heptane, 30/70). Un mélange sous argon de l'intermédiaire obtenu (325 mg ; 0,78 mmol), de triphénylphosphine (42 mg ; 0,156 mmol), d'acétate de potassium (114 mg ; 1,17 mmol ), de bromure de tétrabutylammonium (276 mg ; 0,86 mmol) et d'acétate de palladium (0,078 mmol) est porté au reflux dans de l'acétonitrile anhydre pendant 16 h, puis refroidi à température ambiante et concentré sous pression réduite. Le résidu est purifié par chromatographie à moyenne pression (SiO₂, MeOH/CH₂Cl₂, 5/95) pour donner 80 mg du solide attendu (P_{f} > 250° C).
IR (KBr) :1659, 1734, 3386 cm⁻¹.
RMN ¹H (DMSO-d6, δ) : 0,86 (t, 3H) ; 1,85 (q, 2H) ; 3,07 (d, 1H) ; 3,46 (d, 1H) ; 5,28 (s, 2H) ; 5,39 (d, 1H) ; 5,52 (d, 1H) ; 6,02 (s, 1H) ; 7,43 (s, 1H) ; 7,55 (t, 1H) ; 7,85 (q, 1H) ; 8,01 (d, 1H) ; 8,82 (s, 1H).

### Exemple 4 : (5R)-12-benzyl-5-éthyl-5-hydroxy-4,5,13,15-tétrahydro-1H,3H-oxépino[3',4':6,7]indolizino[1,2-b]quinoléine-3,15-dione (ne fait pas partie de l'invention)

### Etape 4a : 1-(2-aminophényl)-2-phényl-1-éthanone

Une solution de 2-aminobenzonitrile (4,25 g, 36 mmol) dans l'éther diéthylique anhydre (40 ml) à 0° C est traitée sous argon par du chlorure de benzylmagnésium (2M dans le tétrahydrofurane ; 50 ml ; 100 mmol). Le milieu réactionnel est maintenu sous agitation pendant 1h à température ambiante, puis hydrolysé à 0° C par l'ajout d'acide chlorhydrique à 10 %, agité pendant 1 h, et neutralisé avec de la soude. Le mélange résultant est extrait à l'acétate d'éthyle. Les extraits combinés sont lavés à l'eau et avec une solution aqueuse saturée en chlorure de sodium, puis séchés sur sulfate de magnésium et concentrés pour donner 3,5 g du produit désiré, sous la forme d'un solide blanc (P_{f} : 100-101° C).
IR (KBr) : 1469, 1612, 1725 cm⁻¹
RMN ¹H (DMSO-d6, δ) : 4,25 (s, 2H) ; 6,53 (t, 1H) ; 6,74 (d, 1H) ; 7,2-7,35 (m, 8H) ; 7,90 (d, 1H).

### Etape 4b : 4-benzyl-2-oxo-1,2-dihydro-3-quinoléinecarboxylate d'éthyle

Une solution d'amino-cétone (obtenue à l'étape 4a ; 13,5 g ; 16 mmol) et de triéthylamine (3,9 ml, 28 mmol) dans l'acétonitrile (66 ml) est traitée à 10° C au goutte à goutte par du chlorure d'éthylmalonyle (3,64 ml ; 28 mmol). Le milieu réactionnel est agité 16 h à température ambiante, puis traité avec de l'éthoxyde de sodium, obtenu par dissolution de sodium (0,4 g ; 17 mmol) dans l'éthanol (25 ml). Le mélange résultant est agité pendant 16 h à température ambiante, puis de l'eau est ajoutée (200 ml) et on extrait au dichlorométhane (2 x 100 ml). Les extraits combinés sont lavés à l'eau et avec une solution aqueuse saturée en chlorure de sodium, puis séchés sur sulfate de magnésium et concentrés. Le résidu est repris à l'éther éthylique pour donner un précipité qui est recueilli par filtration, séché sous pression réduite à 50° C, pour donner le solide attendu (P_{f} : 230° C).
RMN ¹H (DMSO-d6, δ) : 1,19 (t, 3H) ; 4,17 (s, 2H) ; 4,27 (q, 2H) ; 7,13 (t, 1H) ; 7,15-7,20 (m, 1H) ; 7,20-7,40 (m, 5H) ; 7,49 (t, 1H) ; 7,69 (d, 1H) ; 12,15 (s, 1H).

### Etape 4c : (5R)-12-benzyl-5-éthyl-5-hydroxy-4,5,13,15-tétrahydro-1H,3H-oxépino[3',4':6,7]indolizino[1,2-b]quinoléine-3,15-dione

On applique à la quinolone obtenue selon l'étape 4b les étapes 1f à 1h du mode opératoire de l'exemple 1 ci-dessus. On obtient un solide (P_{f} > 250° C).
IR (KBr) : 1578, 1655, 1751 cm⁻¹.
RMN ¹H (DMSO-d6, δ) : 0,87 (t, 3H) ; 1,87 (q, 2H) ; 3,05 (d, 1H) ; 3,49 (d, 1H) ; 4,65 (d, 1H) ; 4,70 (d, 1H) ; 5,20 (d, 1H) ; 5,25 (d, 1H) ; 5,39 (d, 1H) ; 5,52 (d, 1H) ; 6,06 (s, 1H) ; 7,15 - 7,30 (m, 5H) ; 7,41 (s, 1H) ; 7,67 (t, 1H) ; 7,83 (t, 1H) ; 8,16 (d, 1H); 8,28 (d, 1H).

### Exemple 11 : (5R)-5-éthyl-9,10-difluoro-5-hydroxy-12-phényl-4,5,13,15 -tétrahydro-1H,3H-oxépino [3',4':6,7]indolizino[1,2-b]quinoléine-3,15-dione (ne fait pas partie de l'invention)

### Etape 11a : 6,7-difluoro-2-phényl-4H-benzo[d][3,1]oxazine-4-one

Un mélange d'acide 2-amino-4,5-difluorobenzoïque (3,46 g ; 20 mmol) et de chlorure de benzoyle (56 ml ; 480 mmol) est porté au reflux pendant 16 h, puis versé sur une solution aqueuse saturée en bicarbonate de sodium (200 ml) et agité à 80° C pendant 2 h. Le mélange résultant est extrait au dichlorométhane (2 x 100 ml). Les extraits combinés sont lavés à l'eau et avec une solution aqueuse saturée en chlorure de sodium, puis séchés sur sulfate de magnésium et concentrés sous pression réduite. Le résidu est repris à l'éther éthylique et le précipité ainsi formé est recueilli par filtration, lavé à l'éther éthylique, et séché sous pression réduite pour donner 3,2 g d'un solide blanc (P_{f} : 154° C).
IR (KBr) : 1613, 1657, 3341, 3467 cm⁻¹.
RMN ¹H (DMSO-d6, δ) : 7,5-7,8 (m, 3H) ; 7,8-7,9 (m, 1H) ; 8,1-8,3 (m, 1H).

### Etape 11b: 2-benzoyl-4,5-difluoro-1-phénylcarboxamidobenzène

Une suspension de benzoxazine (obtenue selon l'étape 11a ; 6,78 g ; 26 mmol) dans du dichlorométhane (260 ml) est traitée au goutte à goutte sous argon à -78° C par du bromure de phénylmagnésium (3M dans l'éther éthylique ; 22 ml ; 66 mmol). Le mélange résultant est agité à -78° C pendant 1 h, puis hydrolysé par addition d'une solution aqueuse saturée en chlorure d'ammonium (200 ml) et extrait au dichlorométhane (2 x 100 ml). Les extraits combinés sont lavés à l'eau et avec une solution aqueuse saturée en chlorure de sodium, puis séchés sur sulfate de magnésium et concentrés sous pression réduite. Le résidu repris à l'éther isopropylique donne des cristaux blancs qui sont recueillis par filtration et séchés. On obtient 7,3 g de produit (P_{f} : 58-59° C).
IR (KBr) : 1423, 1537, 1599, 1682 cm⁻¹.
RMN ¹H (DMSO-d6, δ) : 7,4-7,6 (m, 9H) ; 7,69 (d, 2H) ; 7,88 (dd, 1H).

### Etape 11c : 2-amino-4,5-difluorophényl-phénylméthanone

Une solution de d'amino-cétone *N*-benzoylée (obtenue selon l'étape 11b ; 7,3 g ; 21,7 mmol) dans l'acide acétique glacial (300 ml) est traitée avec de l'acide bromhydrique à 48 % (150 ml) et le milieu réactionnel est porté au reflux pendant 10 h. Après refroidissement à température ambiante, le mélange résultant est concentré sous pression réduite, puis repris dans une solution aqueuse saturée en bicarbonate de sodium (200 ml) et extrait à l'acétate d'éthyle (2 x 100 ml). Les extraits combinés sont lavés à l'eau et avec une solution aqueuse saturée en chlorure de sodium, puis séchés sur sulfate de magnésium et concentrés sous pression réduite. Le résidu est repris au pentane et le précipité ainsi formé est recueilli par filtration donner 4 g d'un solide jaune clair (P_{f} : 100-101° C).
IR (KBr) :1514, 1563, 1645, 3372, 3482 cm⁻¹.
RMN ¹H (DMSO-d6, δ) : 6,83 (dd, 1H) ; 7,1-7,4 (m, 3H) ; 7,5-7,7 (m, 5H).

### Etape 11d : (5R)-5-éthyl-9,10-difluoro-5-hydroxy-12-phényl-4,5,13,15-tétrahydro-1H,3H-oxépino[3',4':6,7]indolizino[1,2-b]quinoléine-3,15-dione

On traite l'aminocétone obtenue à l'étape 11c selon une procédure similaire à celle de l'étape 4b. On applique à la quinolone obtenue les étapes 1f à 1h du mode opératoire de l'exemple 1 ci-dessus. On obtient un solide (P_{f} > 250° C).
IR (KBr) :1659, 1734, 3386 cm⁻¹.
RMN ¹H (DMSO-d6, δ): 0,85 (t, 3H) ; 1,80 (q, 2H) ; 3,06 (d, 1H) ; 3,45 (d, 1H) ; 5,00 (d, 1H); 5,35 (d, 1H) ; 5,48 (d, 1H); 6,03 (s, 1H) ; 7,39 (s, 1H) ; 7,55-7,75 (m, 6H) ; 8,24 (dd, 1H).

### Exemple 13 : (5R)-12-butyl-5-éthyl-9,10-difluoro-5-hydroxy-4,5,13,15-tétrahydro-1H,3H-oxépino[3',4':6,7]indolizino[1,2-b]quinoléine-3,15-dione (ne fait pas partie de l'invention)

### Etape 13a : N-(3,4-difluorophényl)acétamide

Un mélange de 3,4-difluoroaniline *(50* ml ; 500 mmol) et de triéthylamine (70 ml ; 500 mmol) dans du dichlorométhane (1,5 l) est refroidi au moyen d'un bain de glace. De l'anhydride acétique (71,5 ml ; 750 mmol) est ajouté goutte à goutte et le mélange réactionnel est agité pendant 1 h à température ambiante. Le mélange obtenu est ensuite lavé séquentiellement avec de l'eau, avec une solution de bicarbonate de sodium à 10 %, et avec une solution aqueuse saturée en chlorure de sodium. La fraction organique, séchée sur du sulfate de sodium, est concentrée sous pression réduite. Le résidu est mis en suspension dans du pentane, filtré et séché sous pression réduite pour donner l'anilide attendu, un solide beige (P_{f} : 126-127,5° C).
RMN ¹H (DMSO-d6, δ) : 2,15 (s, 3H) ; 7,10-7,65 (m, 2H) ; 7,65-8,10 (m, 1H) ; 10,30 (pic large, 1H).

### Etape 13b : 2-chloro-6,7-difluoro-3-quinoléinecarbaldéhyde

A un réactif de Vilsmeyer, obtenu sous argon avec du *N,N*-diméthylformamide anhydre (34 ml ; 440 mmol) refroidi par un bain de glace, traité au goutte à goutte par de l'oxychlorure de phosphore (103 ml ; 1,1 mol), puis agité pendant 0,5 h avant de laisser la température remonter jusqu'à température ambiante, on ajoute l'acétanilide obtenu selon l'étape 13a (32 g ; 220 mmol). Le mélange ainsi obtenu est agité à 70° C durant 16 h, puis refroidi à température ambiante. Le milieu réactionnel est alors coulé goutte à goutte sur un mélange eau-glace (400 ml), et le mélange résultant est agité pendant 2 h. Le précipité obtenu est filtré et lavé à l'eau jusqu'à pH neutre, puis séché sous pression réduite en présence de pentoxyde de phosphore pour donner un solide jaune (P_{f} : 226-229° C).
IR (KBr) : 888, 1061, 1262, 1507, 1691 cm⁻¹.
RMN ¹H (DMSO-d6, δ): 8,17 (dd, 1H) ; 8,39 (dd, 1H) ; 8,97 (d, 1H) ; 10,34 (d, 1H).

### Etape 13c : 2-chloro-6,7-difluoro-3-quinolylméthanol

Une suspension de quinoléine-carbaldéhyde obtenu selon l'étape 13b (9 g ; 39 mmol) dans du méthanol (400 ml) est traitée par du borohydrure de sodium (2 g ; 53 mmol) à température ambiante pendant 0,5 h. L'excès de borohydrure est détruit par de l'acide acétique (2 ml) et le milieu réactionnel est concentré sous pression réduite. Le résidu, repris dans l'acétate d'éthyle (500 ml), est lavé séquentiellement avec une solution aqueuse de bicarbonate de sodium à 10 %, avec de l'eau, et avec une solution aqueuse saturée en chlorure de sodium. La phase organique, séchée sur sulfate de magnésium, est concentrée sous pression réduite. Le résidu est recristallisé dans du 1,2-dichloroéthane pour donner le quinolylméthanol attendu, un solide beige (P_{f} : 166,5-167° C).
IR (KBr) : 871, 1038, 1253, 1513 cm⁻¹.
RMN ¹H (DMSO-d6, δ): 4,67 (d, 2H) ; 5,80 (t, 1H) ; 8,01 (dd, 1H) ; 8,22 (dd, 1H) ; 8,48 (s, 1H).

### Etape 13d : (5R)-5-éthyl-9,10-difluoro-5-hydroxy-4,5,13,15-tétrahydro-1H,3H-oxépino[3',4':6,7]indolizino[1,2-b]quinoléine-3,15-dione

On traite le quinolylméthanol obtenu à l'étape 13c avec du (+)-EHHOPD selon la procédure de l'étape 1h. On obtient un solide blanc.
IR (KBr) : 871, 1261, 1512, 1579, 1654, 1746 cm⁻¹.
RMN ¹H (DMSO-d6, δ) : 0,87 (t, 3H) ; 1,85 (m, 2H) ; 3,08 (d, 1H) ; 3,44 (d, 1H) ; 5,26 (s, 2H) ; 5,39 (d, 2H) ; 5,52 (d, 1H) ; 5,99 (s, 1H) ; 7,39 (s, 1H) ; 8,15 (dd, 1H) ; 8,23 (dd, 1H) ; 8,68 (s, 1H).

### Etape 13e : (5R)-12-butyl-5-éthyl-9,10-difluoro-5-hydroxy-4,5,13,15-tétrahydro-1H,3H-oxépino[3',4':6,7]indolizino[1,2-b]quinoléine-3,15-dione

Le produit de l'étape 13d (100 mg ; 0,25 mmol) est dissous dans un mélange d'eau (1,33 ml) et d'acide sulfurique à 95 % (1 ml). On ajoute à cette solution du sulfate de fer (II) heptahydraté (28 mg ; 0,10 mmol), du valéraldéhyde (0,17 ml ; 1,60 mmol) et l'on refroidit la solution résultante par un bain de glace. Le milieu réactionnel est alors traité au goutte à goutte par du peroxyde d'hydrogène à 30 % (0,38 ml ; 1 mmol), agité pendant 5 h à température ambiante, puis dilué à l'eau (50 ml) et extrait au dichlorométhane (4 x 50 ml). Les extraits combinés sont lavés à l'eau et avec une solution aqueuse saturée en chlorure de sodium, puis séchés sur sulfate de magnésium et concentrés sous pression réduite. Le résidu est purifié par chromatographie à moyenne pression (SiO₂, MeOH/CH₂Cl₂, 5/95) pour donner le solide attendu (Pf > 275° C).
IR (KBr) : 1656, 1748, 3385 cm⁻¹.
RMN ¹H (DMSO-d6, δ) : 0,85 (t, 3H) ; 0,94 (t, 3H) ; 1,47 (q, 2H) ; 1,64 (m, 2H) ; 1,83 (q, 2H) ; 3,05 (d, 1H) ; 3,16 (m, 2H) ; 3,47 (d, 1H) ; 5,27 (s, 2H) ; 5,39 (d, 1H) ; 5,52 (d, 1H) ; 6,05 (s, 1H) ; 7,35 (s, 1H) ; 8,13 (m, 1H); 8,32 (m, 1H).

### Exemple 17 : (5R)-5-éthyl-9,10-difluoro-5-hydroxy-12-(2-triméthylsilyléthyl) -4,5,13,15-tétrahydro-1H,3H-oxépino [3',4':6,7] indolizino[1,2-b]quinoléine-3,15-dione

Le produit de l'étape 13d est traité avec du 3-triméthylsilylpropanal (obtenu suivant Sarkar, T. K., et coll., *Tetrahedron* (1990), vol. **46**, p. 1885) selon une procédure similaire à l'étape 13e pour donner le solide attendu (P_{f} 276° C).
RMN ¹H (DMSO-d6, δ): 0,14 (s, 9H) ; 0,86 (m, 5H) ; 1,83 (q, 2H) ; 3,07 (m, 3H) ; 3,46 (d, 1H) ; 5,26 (s, 2H) ; 5,40 (d, 1H) ; 5,51 (d, 1H) ; 6,06 (s, 1H) ; 7,34 (s, 1H) ; 8,14 (m, 2H).

### Exemple 22 : (5R)-5-éthyl-5-hydroxy-12-(2-triméthylsilyléthyl)-4,5,13,15-tétrahydro-1H,3H-oxépino[3',4':6,7]indolizino[1,2-b] quinoléine-3,15-dione

La (5*R*)-5-éthyl-5-hydroxy-4,5,13,15-tétrahydro-1*H*,3*H*-oxépino[3',4':6,7]indolizino [1,2-*b*]quinoléine-3,15-dione (obtenue selon la procédure décrite dans la demande de brevet PCT WO 97/00876) est traitée avec du 3-triméthylsilylpropanal (obtenu suivant Sarkar, T. K., et coll., *Tetrahedron* (1990), vol.**46**, p. 1885) selon une procédure similaire à celle de l'étape 13e pour donner le solide attendu (P_{f} > 250° C).
IR (KBr) : 1655, 1753, 3420 cm⁻¹.
RMN ¹H (DMSO-d6, δ) : 0,11 (s, 9H) ; 0,88 (t, 3H) ; 0,91 (m, 2H) ; 1,89 (q, 2H) ; 3,07 (d, 1H) ; 3,12 (m, 2H) ; 3,47 (d, 1H) ; 5,25 (s, 2H) ; 5,33 (d, 1H) ; 5,41 (d, 1H) ; 5,54 (d, 1H) ; 6,02 (s, 1H) ; 7,39 (s, 1H) ; 7,73 (t, 1H) ; 7,82 (t, 1H) ; 8,15 (s, 1H).

### Exemple 90 : (5R)-5-éthyl-9-fluoro-5-hydroxy-3,15-dioxo-4,5,13,15-tétrahydro-1H,3H-oxépino[3',4':6,7]indolizino[1,2-b]quinoléin-10-yl trifluorométanesulfonate (ne fait pas partie de l'invention)

La (5*R*)-5-éthyl-9-fluoro-5,10-dihydroxy-4,5,13,15-tétahydro-1*H*,3*H*-oxépino-[3',4':6,7]indolizino[1,2-*b*]quinoléine-3,15-dione (28 mg, obtenue selon la préparation 20 décrite dans la demande de brevet PCT WO 98/28304) en solution dans du DMF anhydre (5 ml) est traitée à 0° C par 1,1 équivalent d'hydrure de sodium, puis avec 1,1 équivalent de N-phenyltrifluorosulfonimide. Le milieu réactionnel est maintenu sous agitation pendant 2 h à température ambiante, puis versé sur de l'eau glacée et extrait à l'acétate d'éthyle. La phase organique est séchée et évaporée puis le résidu est repris à l'éther et recueilli par filtration pour donner le produit attendu.
RMN-¹H (DMSO) : 0,87 (t, 3H) ; 1,86 (q, 2H) ; 3,07 (d, 1H) ; 3,46 (d, 1H) ; 5,29 (s, 1H) ; 5,40 (d, 1H) ; 5,52 (d, 1H) ; 6,04 (s, 1H) ; 7,43 (s, 1H) ; 8,31 (d, 1H) ; 8,66 (d, 1H) ; 8,82 (s, 1H).

### ÉTUDE PHARMACOLOGIQUE DES PRODUITS DE L'INVENTION

### Procédure

Des cellules HT29 d'adénocarcinome de colon humain sont cultivées en mono-couche, à 37° C dans une atmosphère humidifiée contenant 95 % d'air et 5 % de CO₂, dans un milieu essentiel modifié de Earle à 4,5 g/l (Gibco, Paisley, Royaume-Uni); complété avec 10 % de sérum de veau foetal inactivé, 2 mM de glutamine, et 50 µg/ml de gentamycine (Gibco, Paisley, Royaume-Uni).

Environ 2000 cellules sont ensemencées avec le milieu de culture ci-dessus dans les puits d'une microplaque (96 puits, fond plat) et incubées pendant 24 h. Des solutions dans le *N,N*-diméthyl-acétamide (DMA) de chacun des exemples de l'invention, diluées dans le milieu de culture de façon à ce que la concentration finale de DMA soit de 0,1 % (v/v), sont ajoutées aux cultures en plaque, pour obtenir des gammes de concentration finale allant de 1 x 10⁻¹³ à 1 x 10⁻⁵ M, et les cellules sont incubées pendant 72 h.

Le réactif de marquage WST1, (Boehringer Mannheim, Germany) est alors ajouté dans chaque puit à une concentration finale de 9 %, et les cellules sont incubées pendant 2 h à 37° C. Cette étape permet à la déshydrogénase mitochondriale des cellules vivantes de convertir le sel de tétrazolium orangée WST1 en formazan pourpre. Les solutions colorées résultantes sont quantifiées par détection à double faisceaux (450 et 690 nm) à l'aide d'un spectrophotomètre multi-cuves à balayage.

### Résultats

Les résultats consignés dans le tableau qui suit sont exprimés en termes de concentration inhibitrice à 50 % (IC₅₀, en nM), accompagnée d'un intervalle de confiance. On appréciera les activités inhibitrices de la prolifération des cellules HT29 d'adénocarcinome de colon humain obtenues avec les exemples de l'invention, ces activités étant, de façon inattendue, supérieures à l'activité du composé de référence, décrit dans la demande de brevet PCT WO 97/000876 (et correspondant au composé I_{A} dans lequel R₁= Et, R₂ = R₃ = R₄ = R₅ = H, R₆ = H).

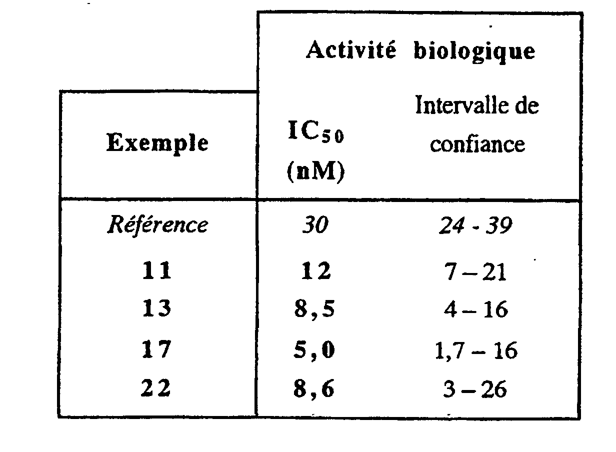

## Revendications

1. Composés de formule générale (I_{A}) 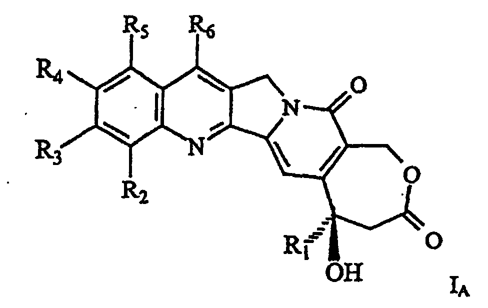 dans laquelle
R₁ représente un radical alkyle inférieur ;
R₂, R₃, R₄ et R₅ représentent, indépendamment, un atome d'hydrogène, un radical halo ou -OSO₂R₁₀;
R₆ représente un radical -(CH₂)ₘSiR₇R₈R₉;
R₇, R₈ et R₉ représentent, indépendamment, un radical alkyle inférieur ;
R₁₀ représente un radical alkyle inférieur éventuellement substitué par un ou plusieurs radicaux halo identiques ou différents, ou aryle éventuellement susbtitué par un ou plusieurs radicaux alkyle inférieur identiques ou différents ;
m est un nombre entier compris entre 0 et 6 ;
étant entendu que lorsque le terme inférieur se réfère à un radical alkyle, cela signifie au plus 6 atomes de carbone;
ou les sels d'addition de ces derniers.

2. Composés selon la revendication 1, dans lesquels R₁ représente un radical éthyle.

3. Composés de formule I_{A} selon la revendication 2 et répondant à l'une des formules suivantes:
(5R)-5-éthyl-9,10-difluoro-5-hydroxy-12-(2-triméthylsilyléthyl)-4,5,13,15-tétrahydro-1*H*,3*H*-oxépino [3',4':6,7]indolizino[1,2-*b*]quinoléine-3,15-dione;
(5*R*)-5-éthyl-5-hydroxy-12-(2-triméthylsilyléthyl)-4,5,13,15-tétrahydro-1*H*,3*H*-oxépino [3',4':6,7] indolizino[1,2-*b*]quiqoléine-3,15-dione.

4. A titre de médicament, un composé de formule I_{A} selon l'une quelconque des revendications 1 à 3, ou un sel pharmaceutiquement acceptable dudit composé.

5. Composition pharmaceutique contenant, à titre de principe actif, au moins un composé selon l'une des revendications 1 à 3.

6. Utilisation d'un composé selon l'une des revendications 1 à 3, pour la préparation de médicaments destinés à inhiber les topoisomérases, et plus particulièrement les topoisomérases de type I ou de type II.

7. Utilisation d'un composé selon l'une des revendications 1 à 3 pour la préparation de médicaments antitumoraux.

8. Utilisation d'un composé selon l'une des revendications 1 à 3 pour la préparation de médicaments antiviraux.

9. Utilisation d'un composé selon l'une des revendications 1 à 3 pour la préparation de médicaments antiparasitaires.

## Claims

1. Compounds of general formula (**I**_{**A**}) 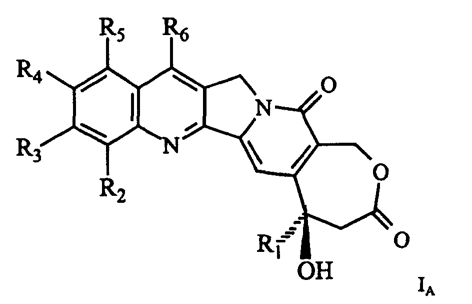 in which
R₁ represents a lower alkyl radical;
R₂, R3, R₄ and R₅ represent, independently, H, halo or -OSO₂R₁₀ ;
R₆ represents a -(CH₂)ₘSiR₇R₈R₉ radical;
R₇, R₈ and R₉ represent, independently, a lower alkyl radical;
R₁₀ represents a lower alkyl radical optionnally substituted by one or more halo radicals identical or different, or an aryl optionnally substituted by one or more lower alkyl radicals identical or different;
m is an integer comprised between 0 and 6 ;
it being understood that the term "lower" when it refers to alkyl radical, means a radical containing up to 6 carbon atoms ;
or the addition salts thereof.

2. Compounds according to claim 1 in which R₁ represents an ethyl radical.

3. Compounds of formula I_{A} according to claim 2 and corresponding to the following formulae:
(5*R*)-5-ethyl-9,10-difluoro-5-hydroxy-12-(2-trimethylsilylethyl)-4,5,13,15-tetrahydro-1*H*,3*H*-oxepino [3',4':6,7]indolizino[1,2-*b*]quinoleine-3,15-dione ;
(5*R*)-5-ethyl-5-hydroxy-12-(2-trimethylsilylethyl)-4,5,13,15-tetrahydro-1*H*,3*H*-oxepino [3',4':6,7] indolizino[1,2-*b*]quinoleine-3,15-dione.

4. As medicament, a compound of formula I_{A} according to any of claims 1 to 3, or a therapeutical acceptable salt thereof.

5. Pharmaceutical composition containing, as an active ingredient, at least one compound as defined in any of claims 1 to 3.

6. Use of the compounds according to any of claims 1 to 3 for the preparation of medicaments intended to inhibit topoisomerases, and more particularly the topoisomerases of type I or of type II.

7. Use of the compounds according to any of claims 1 to 3 for the preparation of antitumoral medicaments.

8. Use of the compounds according to any of claims 1 to 3 for the preparation of antiviral medicaments.

9. Use of the compounds according to any of claims 1 to 3 for the preparation of antiparasitic medicaments.

## Patentansprüche

1. Verbindungen der allgemeinen Formel (I_{A}) 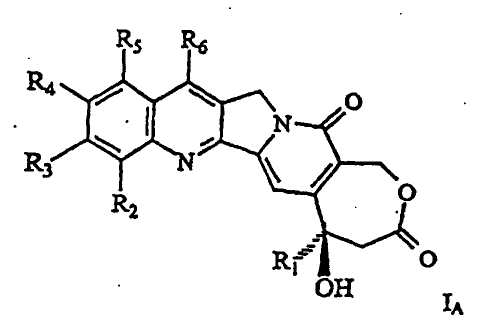 in der
R₁ einen Niederalkylrest darstellt;
R₂, R₃, R₄ und R₅ unabhängig voneinander ein Wasserstoffatom, einen Halogenrest oder einen Rest -OSO₂R₁₀ darstellen; R₆ einen Rest -(CH₂)ₘSiR₇R₈R₉ darstellt;
R₇, R₈ und R₉ unabhängig voneinander einen Niederalkylrest darstellen;
R₁₀ einen Niederalkylrest, der ggf. durch einen oder mehrere gleiche oder verschiedene Halogenreste substituiert ist, oder einen Arylrest darstellt, der ggf. durch einen oder mehrere gleiche oder verschiedene Niederalkylreste substituiert ist;
m eine ganze Zahl zwischen 0 und 6 ist;
wobei der Begriff "Nieder", wenn er sich auf einen Alkylrest bezieht, höchstens 6 Kohlenstoffatome bedeutet;
oder die Additionssalze davon.

2. Verbindungen nach Anspruch 1, in denen R₁ einen Ethylrest darstellt.

3. Verbindungen der Formel I_{A} nach Anspruch 2, die einer der folgenden Formeln entsprechen:
(5*R*)-5-Ethyl-9,10-difluor-5-hydroxy-12-(2-trimethylsilylethyl)-4,5,13,15-tetrahydro-1H,3H-oxepino[3',4':6,7]indolizino[1,2-*b*]chinolin-3,15-dion;
(5*R*)-5-Ethyl-5-hydroxy-12-(2-trimethylsilylethyl)-4,5,13,15-tetrahydro-1*H*,3*H*-oxepino[3',4':6,7]indolizino [1,2-*b*]chinolin-3,15-dion.

4. Eine Verbindung der Formel I_{A} nach einem der Ansprüche 1 bis 3 oder ein pharmazeutisch verträgliches Salz dieser Verbindung in Form eines Medikaments.

5. Pharmazeutische Zusammensetzung, die als Wirkstoff mindestens eine Verbindung nach einem der Ansprüche 1 bis 3 enthält.

6. Verwendung einer Verbindung nach einem der Ansprüche 1 bis 3 für die Herstellung von Medikamenten zur Hemmung der Topoisomerasen und insbesondere der Topoisomerasen vom Typ I oder vom Typ II.

7. Verwendung einer Verbindung nach einem der Ansprüche 1 bis 3 für die Herstellung von antitumoralen Medikamenten.

8. Verwendung einer Verbindung nach einem der Ansprüche 1 bis 3 für die Herstellung von antiviralen Medikamenten.

9. Verwendung einer Verbindung nach einem der Ansprüche 1 bis 3 für die Herstellung von antiparasitären Medikamenten.
